# EUROPEAN PATENT APPLICATION

(11) **EP 1 516 625 A1**
(43) Date of publication of application: **23.03.2005**
(21) Application number: 03292281.7
(22) Date of filing: 16.09.2003
(51) Int. Cl.: A61K 38/02, A61P 25/18, A61P 25/08, A61K 38/18

(54) **Uses of neuronal channels-forming pannexins for therapy and diagnosis in mammals**

(71) Applicant: INSTITUT PASTEUR, 75015 Paris (FR); Université de Heidelberg, 69120 Heidelberg (DE)
(72) Inventor: Monyer, Hannah, c/o Université d'Heidelberg, 69120 Heidelberg (DE); Bruzzone, Roberto, c/o Institut Pasteur, 75009 Paris (FR)
(74) Representative: Warcoin, Jacques

(57) **Abstract**

The present invention is related to the use of neuronal channel-forming pannexins for the manufacture of drugs for preventing and/or treating neurological disorders involving hippocampal pyramidal cells in mammals.

Moreover, the invention concerns the use of pannexins for *in vitro* diagnosing such neurological disorders.

The present invention is also directed to methods for selecting, *in vitro* or *in vivo* using an animal model, compounds useful for preventing and/or treating, in mammals, neurological disorders involving the hippocampal pyramidal cells, by modifying the channel-forming ability of pannexins.

## Description

The present invention relates to the industrial developments in connection with the domain of neurobiology. More precisely, the invention concerns medical and pharmaceutical applications of particular mammalian neuronal proteins.

The present invention is thus related to the use of neuronal channel-forming pannexins for the manufacture of drugs for preventing and/or treating neurological disorders involving hippocampal pyramidal cells in mammals.

Moreover, the invention concerns the use of pannexins for *in vitro* diagnosing such neurological disorders.

The present invention is also directed to methods for selecting, *in vitro* or *in vivo* using an animal model, compounds useful for preventing and/or treating, in mammals, neurological disorders involving the hippocampal pyramidal cells, by modifying the channel-forming ability of pannexins.

Gap junctions are collections of intercellular channels that, in vertebrates, are formed by connexins, a multi-gene family of which 20 members have been identified in humans (1). It is generally accepted that gap junctions between neurons represent the anatomical substrate of electrical synapses (reviewed in 2, 3). Although the incidence of electrical coupling relative to chemical synapses in the adult is relatively low, several studies have demonstrated that different types of interneurons of the hippocampus and neocortex communicate via electrical synapses in a cell-specific manner (4-11). Therefore, this additional form of intercellular communication appears to be more widespread than previously imagined and delineates independent networks of coupled cells. In this respect, electrical synapses have emerged as a common mechanism for synchronizing neuronal ensembles at different frequency bands, which have been proposed to underlie a variety of cognitive processes (e.g., perception, learning, and memory).

More precisely, besides the undisputed role of chemical transmission in network oscillations, both computer simulations and electrophysiological recordings have recently emphasized a key role for electrical synapses in the generation of synchronous activity in the hippocampus and neocortex (6, 12-17). Accordingly, the identification of connexin36 (Cx36) as the main neuronal connexin expressed in several areas of the brain (18) suggested that it may be an important component of gap junctions involved in the synchronization of large-scale neuronal networks. This possibility has been directly tested in mice with a targeted ablation of Cx36, which exhibit impaired electrical coupling in several brain regions (15, 19-23). Loss of this gap junction protein abolishes electrical coupling between hippocampal interneurons and disrupts gamma frequency network oscillations *in vitro* and *in vivo* (15, 24). The specificity of this impairment was indicated by the finding that high frequency rhythms in hippocampal pyramidal cells are unaffected by the lack of Cx36 (15).

These observations raise two possibilities: either a different connexin is specifically deployed throughout the pyramidal cell network or, alternatively, another class of molecules expressed in the mammalian brain forms electrical junctions between pyramidal cells. The latter hypothesis has received theoretical support from the discovery, in the database, of a novel family of genes encoding proteins for which the name "pannexins" has been proposed (25).

Pannexins are known to share structural features with gap junction proteins of invertebrates (innexins) and vertebrates (connexins) (25).

As shown for the first time in the context of the present invention, pannexins can form gap junction channels and, therefore, contribute to electrical communication in the nervous system of mammals. Indeed, it appears that pannexins form intercellular channels that allow communication between neurons, thus making a novel class of electrical synapses exhibiting some specific features compared to the channel-forming proteins characterized so far.

On the one hand, in contrast to connexins, the other class of proteins forming intercellular channels in mammals, which are mainly targeted to dendrites and form inter-dendritic electrical synapses, pannexins may also be axonally targeted and form axo-axonal gap junctions.

On the other hand, as shown herein, pannexins are interestingly the first proteins with gap junction-forming ability to be localized in the pyramidal cells of the hippocampus of mammals.

On the basis of their cellular distribution in the brain (see the detailed description below), and their aforementioned specific features, pannexins are thought to underlie neurological disorders involving hippocampal pyramidal neurons.

Therefore, thanks to their specific features, pannexins represent an advantageous tool for the development of drugs that will modify their channel forming properties.

Thus, the first aspect of the present invention concerns the use of at least one neuronal channel-forming pannexin, or at least one biologically active fragment thereof, or at least one biologically active derivative thereof, for the manufacture of a drug for preventing and/or treating, in a mammal, a neurological disorder involving the hippocampal pyramidal cells.

The "use" as mentioned above encompasses any use of pannexins or biologically active fragments or derivatives thereof, for pharmaceutical purposes, which means the use of pannexins or biologically active fragments or derivatives thereof, either directly (e.g., the use of pannexins themselves as drugs), or indirectly. Examples of indirect uses of pannexins are the use as screening tools for selecting compounds which may be used as drugs, or the use as starting materials for obtaining such compounds, via modification, transformation, etc..., of the pannexin structure.

The terms and expressions « pannexins », « gap junction proteins », « channel-forming proteins », « gap junction pannexins », « channel-forming pannexins » refer to the same proteins and can be used interchangeably.

For purposes of the invention, the terms "peptides", "proteins" and "polypeptides" are synonymous. A "peptide" is a molecule comprised of a linear array of amino acid residues connected to each other in the linear array by peptide bonds. Such linear array may optionally be cyclic, i.e., the ends of the linear peptide or the side chains of amino acids within the peptide may be joined, e.g., by a chemical bond. Such peptides according to the invention may include from about three to about 500 amino acids, and may further include secondary, tertiary or quaternary structures, as well as intermolecular associations with other peptides or other non-peptide molecules. Such intermolecular associations may be through, without limitation, covalent bonding (e.g., through disulfide linkages), or through chelation, electrostatic interactions, hydrophobic interactions, hydrogen bonding, ion-dipole interactions, dipole-dipole interactions, or any combination of the above.

In addition, certain preferred peptides according to the invention comprise, consist essentially of, or consist of an allelic variant of pannexin. As used herein, an "allelic variant" is a peptide having from one to two amino acid substitutions from a parent peptide, but retaining the biological activity of interest of the parent peptide.

"Retaining the biological activity of interest of the parent peptide" means herein retaining the ability of pannexin to contribute to channel formation.

Peptides according to the invention can be conveniently synthesized using art recognized techniques.

Preferred peptidomimetics retain the biological activity of the parent peptide, as described above. As used herein, a "peptidomimetic" is an organic molecule that mimics some properties of peptides, preferably their biological activity. Preferred peptidomimetics are obtained by structural modification of peptides according to the invention, preferably using unnatural amino acids, D amino acid instead of L aminoacid, conformational restraints, isosteric replacement, cyclization, or other modifications. Other preferred modifications include without limitation, those in which one or more amide bond is replaced by a non-amide bond, and/or one or more amino acid side chain is replaced by a different chemical moiety, or one of more of the N-terminus, the C-terminus or one or more side chain is protected by a protecting group, and/or double bonds and/or cyclization and/or stereospecificity is introduced into the amino chain to increase rigidity and/or binding affinity.

Still other preferred modifications include those intended to enhance resistance to enzymatic degradation, improvement in the bioavailability, and more generally in the pharmacokinetic properties, compared to a parent pannexin peptide.

All of these variations are well known in the art. Thus, given the peptide sequences of pannexin, those skilled in the art are enabled to design and produce peptidomimetics having binding characteristics similar to or superior to such peptides.

The peptides used in the therapeutic method according to the present invention may also be obtained using genetic engineering methods.

A person skilled in the art will refer to the general literature to determine which appropriate codons may be used to synthetize the desired peptide.

A method that allows a person skilled in the art to select *in vitro*, and optionally to purify a biologically active derivative that exhibits an agonist or an antagonist biological activity of a pannexin is described hereunder. According to this method, the selection of said biologically active derivative is performed via determining the changes induced by this candidate compound, such as the channel-forming ability involving a pannexin.

A biologically active derivative of a pannexin may be a protein, a peptide, a hormone, an antibody or a synthetic compound. A definition of the term "compound" is given hereafter.

In the context of the present invention, a "mammal" is an animal or a human. Preferably, by "mammal", it is meant herein a human.

As used herein, the terms "neurological disorder involving the hippocampal pyramidal cells" mean any "disorder" (or "disease" or "trouble") related to malfunctions or impairments or disruptions of electrical coupling between hippocampal pyramidal neurons. Since this electrical coupling is responsible for synchronizing neurons, said malfunctions or impairments or disruptions affect the following biological processes: ultra-fast oscillations in the hippocampus, memory storage, higher cognitive functions (e.g., perception, learning, memory), olfaction, and vision. Thus, a "neurological disorder involving the hippocampal pyramidal cells" as referred to herein is preferably selected from epilepsy, schizophrenia, memory disorders, pain disorders, visual deficits, visual acuity, odor discrimination, and olfaction deficits.

The second aspect of the present invention is directed to the use of at least one neuronal pannexin for *in vitro* diagnosing, in a mammal, a neurological disorder involving the hippocampal pyramidal cells.

Such a use for *in vitro* diagnostic entails advantageously at least one of the following :
- mapping of a specific disorder to the chromosome region where the pannexin gene is located;
- sequencing of said gene;
- identification of at least one mutation comprised therein;
- testing the functional effects of said mutation;
- establishing a genotype/phenotype correlation.

In a particular embodiment, said *in vitro* diagnostic comprises at least:
a) sequencing a pannexin gene in a mammal suspected to have a neurological disorder involving the hippocampal pyramidal cells; and
b) identifying at least one mutation responsible for the lack of production of pannexin, or for the production of an inactive pannexin, in said mammal.

According to a third aspect, the present invention relates to methods for *in vitro* selecting a compound useful for preventing and/or treating, in a mammal, a neurological disorder involving the hippocampal pyramidal cells. The selection is based upon the fact that said compound is capable of modifying the channel-forming ability involving a pannexin.

By "modifying or modulating the channel-forming ability of a pannexin" or "modifying or modulating the channel-forming ability involving a pannexin", it is meant that said channel-forming ability is either induced (equivalents of "induced" being herein "increased", "promoted", "enhanced", and "stimulated"), or inhibited (equivalents of "inhibited" being "reduced", "decreased", "suppressed", and "blocked"). This may reflect, for instance, (i) an increase or decrease in expression or in activity of the pannexin polynucleotides or proteins; or (ii) a change in the amount of said polynucleotides or proteins, in the cellular distribution thereof, in the level of expression thereof, in the type of activity thereof.

In a first embodiment, such a method comprises at least:
a) measuring the channel-forming ability of a pannexin in the absence of any compound (P0);
b) measuring the channel-forming ability of said pannexin in the presence of a compound (P1);
c) comparing P0 and P1; and
d) if P1 is significantly different from P0, selecting said compound.

On the one hand, in step d), if P1 is significantly greater than P0, the selected compound is an agonist of said pannexin.

By « agonist », it is meant herein a compound capable of restoring or increasing the channel-forming ability and/or the amplitude and/or kinetics of the membrane currents involving a pannexin.

On the other hand, in step d), if P1 is significantly lower than P0, the selected compound is an antagonist of said pannexin.

An "antagonist" is herein a compound capable of inhibiting or decreasing the channel-forming ability and/or the amplitude and/or kinetics of the membrane currents involving a pannexin.

A "compound" herein refers to any type of molecule, biological or chemical, natural, recombinant or synthetic. For instance, such a compound may be a nucleic acid (e.g., an antisense or sense oligonucleotide including an antisense RNA), a peptide, a fatty acid, an antibody, a polysaccharide, a steroid, a purine, a pyrimidine, an organic molecule, a chemical moiety, and the like. Also encompassed by the term "compound" are fragments, derivatives, structural analogs or combinations of the above. In particular, the biologically active fragments or derivatives of pannexins, as defined above, are also encompassed by the term "compound".

Methods for measuring channel-forming ability of proteins are well-characterized in the art. For instance, the skilled artisan, relying on the detailed description below, can perform the experimental procedure recited therein.

In a second embodiment, the method of the invention allows the selection of a compound of interest, based on its specific modulatory action on pannexins. In this respect, such a method for *in vitro* selecting a compound useful for preventing and/or treating, in a mammal, a neurological disorder involving the hippocampal pyramidal cells, said compound being capable of specifically modifying the channel-forming ability of a pannexin, without modifying the channel-forming ability of a connexin, comprises at least:
a) measuring the channel-forming ability of each of said pannexin (P0) and said connexin (C0) in the absence of said compound;
b) measuring the channel-forming ability of each of said pannexin (P1) and said connexin (C1) in the presence of said compound;
c) comparing P0 and P1, and C0 and C1; and
d) if P1 is significantly different from P0, and if C1 is not significantly different from C0, selecting said compound.

Yet in this embodiment, the selected compound is either an agonist or an antagonist as defined above.

In both embodiments described above, the method of selection advantageously further comprises purifying said selected compound.

Purification may be performed using standard techniques that are well known by the person skilled in the art.

According to a fourth aspect, the present invention is directed to an animal model for *in vivo* selecting a compound useful for preventing and/or treating, in a mammal, a neurological disorder involving the hippocampal pyramidal cells.

As indicated above, the selection is based upon the fact that said compound is capable of modifying the channel-forming ability involving a pannexin in said animal model.

In a particular embodiment, such an animal model is constructed by introducing at least one mutation in a pannexin gene of an animal, said mutation being responsible for the lack of production of pannexin, or for the production of an inactive pannexin, in said animal model.

The present invention is illustrated, while not being limited, by the following figures:

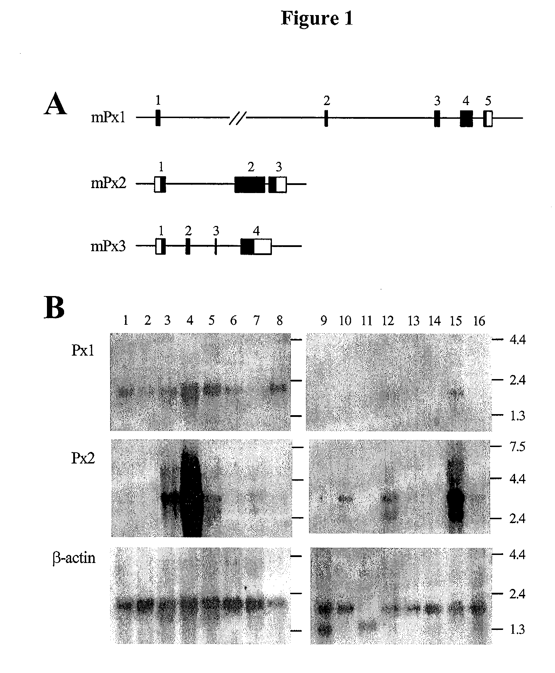

**Fig. 1. Gene organization and mRNA expression in rodents.**
   **(A)** The loci of the three pannexins (Px) in the mouse genome, indicating their exon (numbered boxed regions) and intron structure, are displayed. Within each exon, nucleotides contributing to the presumed protein sequence for each pannexin are shaded.
   **(B)** Northern blot analysis performed on rat polyA+ RNA.
   Lanes 1-16: adrenal gland, bladder, eye, spinal cord, thyroid, stomach, prostate, large intestine, testis, kidney, skeletal muscle, liver, lung, spleen, brain, heart.

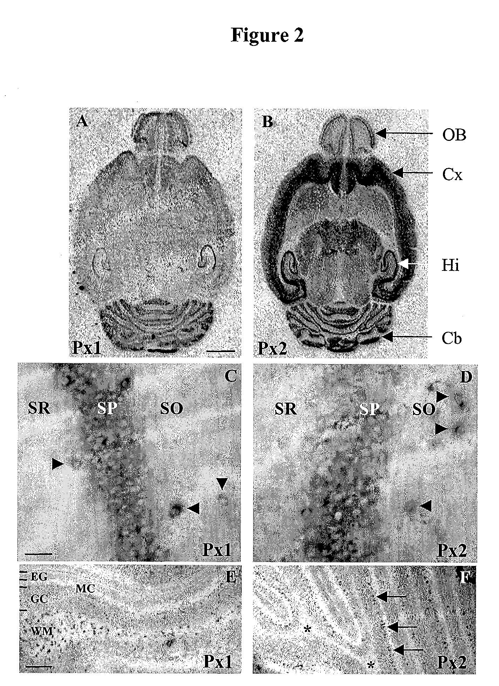

**Fig. 2. Expression of Px1 and Px2 mRNA in the brain.**
   **(A-B)** Distribution of transcripts encoding Px1 and Px2.
      OB: olfactory bulb; Cx: cortex; Hi: hippocampus; Cb: cerebellum.
      Scale bar is 2.5 mm.
   **(C-F)** Non-radioactive *in situ* hybridization.
      (C) and (E): expression of Px1; (D) and (F): expression of Px2.
      SP: *stratum pyramidalis;* SO: *stratum oriens;* SR: *stratum radiatum;* WM: white matter; EG: external granule cell layer; MC: molecular cell layer; GC: granule cell layer.
      Scale bars are 50 µm (C-D) and 250 µm (E-F).

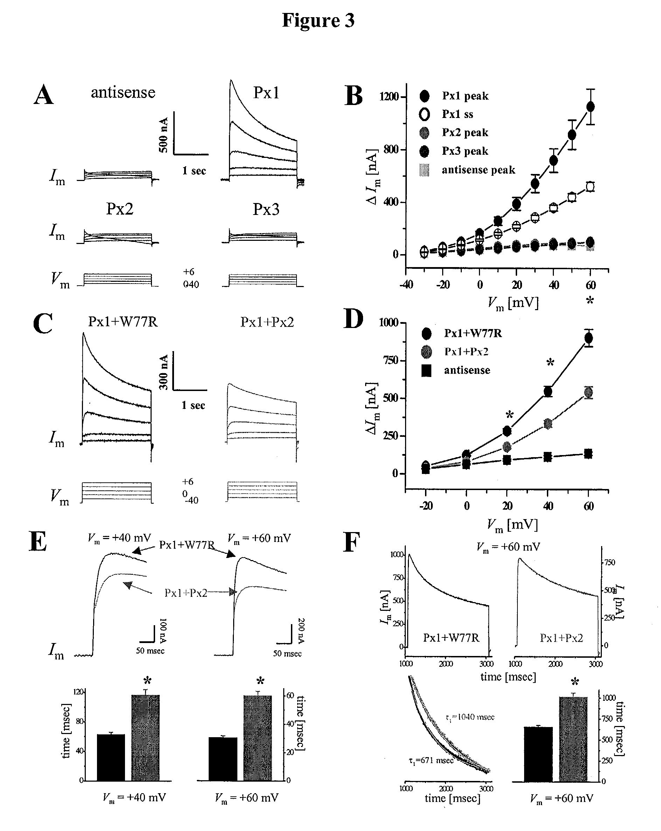

**Fig. 3. Functional expression of pannexins in single *Xenopus* oocytes.**
   **(A)** Whole-cell membrane currents (*I*ₘ) from single oocytes.
      For clarity, representative traces are shown only in 20 mV increments.
   **(B)** Current-voltage relationships.
      Current-voltage relationships were determined for oocytes injected with either antisense oligonucleotides, or Px1, Px2, and Px3 RNAs plus antisense. Results are shown as mean±SEM from at least 8 independent experiments. Antisense (n=45); Px1 (n=80); Px2 (n=46); Px3 (n=41).
   **(C-F)** Functional interaction of Px1 and Px2 proteins.
      Antisense-treated oocytes were co-injected with Px1 RNA together with equal amounts of RNAs encoding either Px2 or the W77R mutation of human Cx26, which is devoid of functional activity (31).
   (C-D) Results are shown as mean±SEM from 4 independent experiments. Antisense (n=39); Px1+W77R (n=60); Px1+Px2 (n=67).
   (E) The lower panels show the mean±SEM from 3 independent experiments for Px1+W77R (n=45) and Px1+Px2 (n=50); **P*<0.001.
   (F) The lower right panel illustrates the mean±SEM from 3 independent experiments, for Px1+W77R (n=44) and Px1+Px2 (n=41); **P*<0.001.

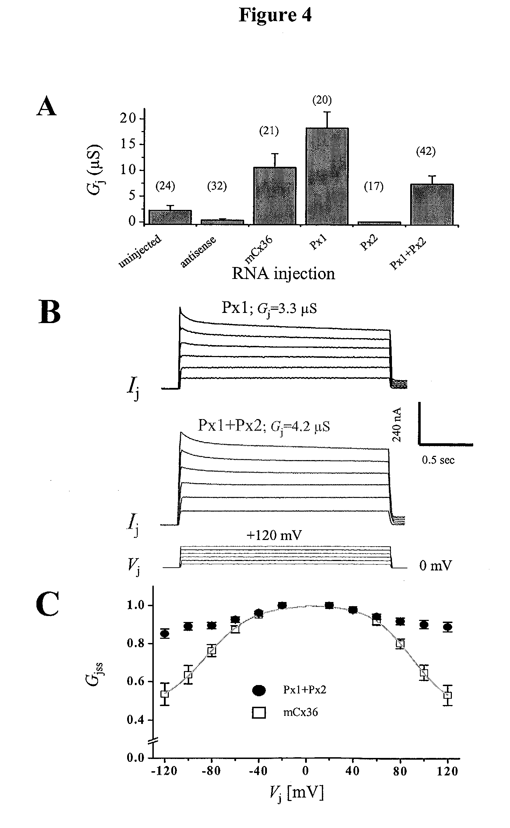

**Fig. 4. Functional expression of pannexins in paired oocytes.**
   (A) Results are shown as the mean±SEM of the indicated number of oocyte pairs from 4-5 independent experiments. *G*ⱼ values recorded from oocytes expressing the neuronal mouse connexin36 (mCx36) are shown for comparison.
   (B) Px1 and Px1+Px2 intercellular channels.
   (C) Results are shown as the mean±SEM of 7-12 pairs (from 4 independent experiments) whose *G*ⱼ was 3.2±0.8 µS and 4.8±1.1 µS for mCx36 and Px1+Px2, respectively.

The present invention will be better understood in the light of the following detailed description of experiments, including examples. Nevertheless, the skilled artisan will appreciate that this detailed description is not limitative and that various modifications, substitutions, omissions, and changes may be made without departing from the scope of the invention.

### I. EXPERIMENTAL PROCEDURES

### I-1-Molecular cloning and mRNA distribution.

cDNA clones were obtained by screening a rat hippocampal cDNA library (postnatal day 15) with [α-³²P] end labeled oligonucleotides complementary to nucleotides (nt) 181-225 and 316-360 of the mouse Px1 open reading frame (ORF), and to nt 199-243 and 334-378 of the human Px2 ORF (25). A probe for Px3 was generated by PCR using the oligonucleotide pair derived from nt 569-592 and 1059-1082 of the rat Px3 ORF, identified in the database. The tissue distribution of pannexin gene expression was investigated by reacting blots containing rat polyA+ RNA (Rat MTN Blot I and II, catalog #7764-1 and 7795-1, respectively; Clontech, Palo Alto, CA) with [α-³²P] dCTP labeled probes derived either from the entire ORF of Px1 and Px2, or with a fragment derived from the β-actin transcript (supplied along with the blots).

For Northern blot analysis, the two filters were hybridized with probes for each of the three pannexins and exposed for 16 hrs at -70°C (Fig. 1 B).

Radioactive (26) and non-radioactive (27) *in situ* hybridization experiments were performed essentially as described previously.

[α-³⁵S] dATP end labeled oligonucleotides corresponded to nt 181-225 and 334-378 of the mouse Px1 and human Px2 coding sequence, whereas the entire rat ORF was used to generate digoxygenin-labeled sense and antisense riboprobes.

The distribution of transcripts encoding Px1 and Px2 was determined by radioactive *in situ* hybridization in horizontal brain sections obtained from rats at postnatal day 15 (Fig. 2, A-B).

### I-2- Functional expression in Xenopus oocytes.

The coding sequence of each pannexin was subcloned into the pBSxG expression vector (28). *In vitro* transcription, preparation of *Xenopus* oocytes, biochemical analysis and RNA injection were performed as described elsewhere (29). Metabolic labeling of oocytes indicated that all three pannexin RNAs directed the synthesis of specific polypeptide bands, whose electrophoretic mobility was similar to that of the *in vitro* translated constructs (not shown).
For physiological analysis, cells were injected with a total volume of 40 nl of either an antisense oligonucleotide (3 ng/cell) to suppress the endogenous *Xenopus* Cx38 (30), or a mixture of antisense plus the specified RNA (20-80 ng/cell). The ability of pannexins to form hemi-channels was assessed in single oocytes 2-4 days after RNA injection, using a two-electrode voltage-clamp.
Whole-cell membrane currents (*I*ₘ) were measured from single oocytes co-injected with pannexin RNAs and an oligonucleotide antisense to *Xenopus* Cx38 (Fig. 3A). Cells were initially clamped at a membrane potential (*V*ₘ) of -40 mV and depolarizing steps lasting 2 sec were applied in 10 mV increments up to +60 mV (bottom traces).
In Fig. 3B, peak current values above holding currents (Δ*I*ₘ) were calculated and plotted as a function of *V*ₘ. Mean values from Px1-injected cells were significantly different (*P*<0.01) from those of control oocytes starting at a *V*ₘ of -10 mV. For Px1 steady-state currents (open circles), values recorded for 20 msec at the end of the pulse were averaged and plotted as above.

To investigate whether Px1 and Px2 could functionally interact, oocytes were co-injected with Px1 RNA (40-80 ng/cell) together with the specified amounts of RNAs encoding either Px2 or the W77R mutation of human Cx26, which is devoid of functional activity (31). To analyze whether pannexins formed intercellular channels, oocytes were stripped of the vitelline envelope 1-2 days after RNA injection, and manually paired in homotypic configuration (same construct in both oocytes) for 24-48 hr before measuring junctional conductance with a dual voltage clamp (Fig. 4). The setup, hardware and software used for electrophysiological measurements and data analysis were as previously described (29, 32).
Both cells of a pair were initially clamped at -40 mV, and alternating pulses of ±10-20 mV were imposed to one cell (Fig. 4A). The current delivered to the cell clamped at -40 mV during the voltage pulse is equal in magnitude to the junctional current and can be divided by the voltage to yield the value of junctional conductance (*G*ⱼ).
In Fig. 4B, junctional currents (*I*ⱼ) were recorded from oocyte pairs in response to (*V*ⱼ steps of opposite polarity (bottom traces) applied, from a holding potential of -40 mV, in 20 mV increments.
In Fig. 4C, the steady-state junctional conductance *G*ⱼₛₛ was measured at the end of the *V*ⱼ step and normalized to the values recorded at ±20 mV; Px1 +Px2 (filled circles) and mCx36 (open squares). Data describing the *G*ⱼ/*V*ⱼ relationship were fit (smooth cyanide lines) to a Boltzmann equation, whose parameters were in agreement with those previously reported (43, 44).

### I-3- Statistical analysis.

Results are shown as mean±SEM. An independent experiment is defined as a series of data obtained with oocytes isolated from one animal. Comparisons between two populations of data were made using the Student's unpaired t-test. P values of 0.01 or less were considered to be significant.

### I-4- Database accession numbers.

The rat pannexin cDNAs were assigned the following accession numbers: AJ557015 (Px1); AJ557016 (Px2); AJ557017 (Px3).

The human pannexins Px1, Px2, and Px3 were assigned accession numbers NP_056183, NP_443071, and NP_443191, respectively.

### II. RESULTS

### II-1- Structure and organization of the pannexin genes.

Analysis of the cDNA sequences for Px1, Px2 and Px3 identified open reading frames (ORFs) encoding proteins with calculated molecular mass of 48,072, 73,270 and 44,976 daltons, respectively. The sequences of all three proteins predict, like for connexins, four transmembrane domains and cytoplasmic amino- and carboxy termini. A hallmark of gap junction-forming proteins is the presence of conserved, regularly spaced cysteine residues located on the two extracellular loops. Whereas the connexins contain three such residues, pannexins contain only two, thus resembling in this respect innexins, the invertebrate constituents of intercellular channels (33). A comparison of the cDNAs to the mouse genomic sequence (obtained from the Ensembl database; http://www.ensembl.org) resulted in the determination of the exon-intron structure of the three mouse pannexin genes (Fig. 1A). Considerable variability was found in the organization and length of the three gene loci, the protein coding regions could be assigned to five, three, and four exons respectively, for the Px1, Px2, and Px3 genes.

### II-2- Distribution of pannexin mRNA.

Northern blots indicated that Px1 and Px2 transcripts were co-expressed in many tissues including eye, thyroid, prostate, kidney, liver and CNS (Fig. 1 B). Px1 probe hybridized to a 2.2-kb mRNA that was detectable in several organs including spinal cord and brain (Fig. 1 B). The 3.5-kb Px2 was most abundant in spinal cord and brain and was also present in other organs (Fig. 1 B). A less prominent 2.5-kb transcript was observed in some organs. By contrast, Px3 transcripts could only be detected in the skin, which was found, by RT-PCR, to be devoid of Px1 and Px2 mRNA (not shown).
*In situ* hybridization studies demonstrated widespread expression of both transcripts in many brain regions, including cortex, striatum, olfactory bulb, hippocampus, thalamus, cerebellum. Fig. 2, A and B, illustrate a partially overlapping expression profile and indicate that the transcripts encoding Px1 and Px2 are abundant in the olfactory bulb, cortex, hippocampus, and cerebellum. No signal was detected in parallel competition experiments with an excess of unlabeled probe (not shown).

Upon closer inspection at the cellular level, a differential distribution of Px1 and Px2 mRNA was apparent. In hippocampus, for example, both Px1 (Fig. 2C) and Px2 (Fig. 2D) were expressed in the pyramidal cell layer and in individual neurons (arrowheads) in the *stratum oriens* and *stratum radiatum.* Based on their location, the scattered neurons (NeuN positive, data not shown) can be inferred to be GABAergic interneurons. By contrast, in the cerebellum, Px1 expressing cells (Fig. 2E) were abundant in the white matter where Px2 expression was absent (Fig. 2F, asteriks; note, however, the high Px2 labeling in the Purkinje cell layer in Fig. 2F, arrows). The labeling of Px1 expressing cells in the white matter was not restricted to the cerebellum but was also observed in other white matter structures (e.g. *corpus callosum, fimbria fornix*), which, similarly, were also devoid of Px2 expression (not shown).

No staining was obtained with sense probes (not shown).

### II-3- Functional expression in single Xenopus oocytes.

Large, voltage-activated outward currents were consistently induced when oocytes expressing Px1 were stepped to voltages greater than -20 mV (Fig. 3A-B). At large positive potentials, Px1 currents reached a peak within 30-60 msec of the imposition of the voltage step and then declined slowly, this rectification becoming more pronounced with increasing positive potentials. By contrast, neither Px2 nor Px3 induced membrane currents above those recorded in controls (Fig. 3A-B). Furthermore, incubation of oocytes for 10-30 min in carbenoxolone completely suppressed Px1 currents (peak amplitudes at +60 mV were 1189±170 and 255±47 nA for control medium and 30 µM carbenoxolone, respectively; n=4) and this effect was fully reversible (peak amplitude at +60 mV after a 30 min recovery period in control medium was 963±239 nA; n=4).
Since the *in situ* hybridization studies revealed co-expression of Px1 and Px2, our subsequent investigations entailed a more detailed functional analysis of these two proteins. To test whether they could form heteromeric channels, currents were recorded from oocytes co-expressing Px1 with Px2 (40-80 ng of RNA/cell) and were found to be significantly reduced with respect to those measured from cells that had been injected with the same amount of Px1 RNA (not shown). To exclude the possibility that this behavior was merely due to overloading of the synthetic machinery given the difference in the total amount of RNA injected, oocytes were injected with equal amounts of RNA for Px1 and the W77R mutation of human Cx26 (40-80 ng each/cell), which is devoid of functional activity (31). These experiments showed a reduction in current amplitude, suggesting that Px1 +Px2 form channels that are different from those composed of Px1 alone (Fig. 3C-D). As illustrated, Δ*I*ₘ recorded from Px1 +Px2 expressing oocytes was significantly less (**P*<0.001) than that measured from Px1+W77R cells.
Given that Px2 expression in the brain appears to be much stronger than Px1, we also tested whether Px2 could simply function as a dominant negative partner by co-injecting Px1 and Px2 RNAs at a 1:5 ratio (40:200 ng/cell for Px1:Px2, respectively). Current amplitudes recorded at +60 mV were similar, irrespective of whether Px1 and Px2 were injected at a ratio of 1:1 (591±40 nA; n=27) or of 1:5 (517±45 nA; n=20), further indicating that they both interact and form functionally heteromeric channels. Interestingly, a decrease in current amplitude was not observed when voltage-activated currents were measured from oocytes receiving RNAs for Px1 and Px3, which are not co-expressed in rat tissues (not shown). Moreover, following the imposition of a voltage step, Px1 +Px2 channels reached peak currents with a significant delay compared to Px1 expressing cells (Fig. 3E), which could result from slower opening or slower closing or both. In Fig. 3E, oocytes were depolarized to +40 mV (top left traces) and +60 mV (top right traces) from a holding potential of -40 mV. Peak currents were reached with a significant delay following the imposition of the voltage step (32 and 68 msec at +40 mV and 62 and 96 msec at +60 mV, for Px1+W77R and Px1+Px2, respectively).
Finally, as illustrated in Fig. 3F, analysis of the kinetics of channel closure at the more positive membrane potentials, revealed that currents recorded from Px1+Px2 expressing cells, presumably reflecting heteromeric hemi-channels, gated more slowly than those measured from oocytes injected with Px1+W77R RNA, presumably reflecting homomeric Px1 hemi-channels. Px2 slows the kinetics of voltage-dependent closure of Px1 hemi-channels. Cells were depolarized to +60 mV from a holding potential of -40 mV (top panels). The time-dependent decline in *I*ₘ was well fit by a first order exponential decay function (lower left panel; cyanide line superposed to the re-scaled current traces shown above).

### II-4- Functional expression in paired Xenopus oocytes.

Px1 alone and in combination with Px2 induced the assembly of intercellular channels, whereas Px2 alone failed to do so (Fig. 4A). As illustrated, pairs of uninjected cells from the different batches of oocytes developed a variable level of junctional currents that exhibited the well-known voltage-dependent gating of endogenous Cx38 (42), whereas antisense controls showed negligible coupling, indicating that endogenous currents had been suppressed. It should be noted that intercellular channels were consistently detected only from batches of oocytes in which a robust junctional conductance was recorded with homotypic pairs expressing either mouse Cx36 (Fig. 4A) or human Cx26 wild-type (not shown), which served as positive controls. In this series of experiments, 20 out of 23 Px1 pairs and 36 out of 42 Px1+Px2 pairs were coupled. As shown in Fig. 4B, both Px1 and Px1+Px2 pairs displayed a remarkable insensitivity to transjunctional potentials of opposite polarities (Vⱼ). Thus, with a driving force ≤ ±60 mV, junctional currents varied linearly with voltage (Fig. 4B) whereas, at higher transjunctional potentials, the conductance of Px1+Px2 channels displayed only a very modest reduction (∼15% ) of the initial values (Fig. 4C), similar to what reported for crayfish septate junctions (34) and human Cx31.9 (32). In Fig. 4C, the plot shows the relationship of *V*ⱼ to steady-state junctional conductance (*G*ⱼₛₛ). Because of the much larger non-junctional currents that were present in Px1 homotypic pairs, reliable *G*ⱼₛₛ/*V*ⱼ plots with the complete polarization paradigm were difficult to obtain.
Although it has been reported that junctional currents measured in insect cells are sensitive to changes in membrane potential (35), the relative voltage insensitivity of pannexin intercellular channels with polarization of one cell is a strong indication that polarization of both cells is not likely to affect significantly junctional conductance.

### REFERENCES

1. Willecke, K., Eiberger, J., Degen, J., Eckardt, D., Romualdi, A., Guldenagel, M., Deutsch, U. & Sohl, G. (2002) *Biol Chem* **383,** 725-37.
2. Bennett, M.V. (2000) *Brain Res Brain Res Rev* **32,** 16-28.
3. Galarreta, M. & Hestrin, S. (2001) *Nat Rev Neurosci* **2,** 425-33.
4. Katsumaru, H., Kosaka, T., Heizmann, C.W. & Hama, K. (1988) *Exp Brain Res* **72,** 363-70.
5. Galarreta, M. & Hestrin, S. (1999) *Nature* **402,** 72-5.
6. Gibson, J.R., Beierlein, M. & Connors, B.W. (1999) *Nature* **402,** 75-9.
7. Beierlein, M., Gibson, J.R. & Connors, B.W. (2000) *Nat Neurosci* **3,** 904-10.
8. Tamas, G., Buhl, E.H., Lorincz, A. & Somogyi, P. (2000) *Nat Neurosci* **3,** 366-71.
9. Venance, L., Rozov, A., Blatow, M., Burnashev, N., Feldmeyer, D. & Monyer, H. (2000) *Proc Natl Acad Sci USA* **97,** 10260-5.
10.Meyer, A.H., Katona, I., Blatow, M., Rozov, A. & Monyer, H. (2002) J *Neurosci* **22,** 7055-64.
11.Blatow, M., Rozov, A., Katona, I., Hormuzdi, S.G., Meyer, A.H., Whittington, M. A., Caputi, A. & Monyer, H. (2003) *Neuron* **38,** 805-17.
12.Ylinen, A., Bragin, A., Nadasdy, Z., Jando, G., Szabo, I., Sik, A. & Buzsaki, G. (1995) *J Neurosci* **15,** 30-46.
13.Draguhn, A., Traub, R.D., Schmitz, D. & Jefferys, J.G. (1998) *Nature* **394,** 189-92.
14.Traub, R.D. & Bibbig, A. (2000) *J Neurosci* **20,** 2086-93.
15.Hormuzdi, S.G., Pais, I., LeBeau, F.E., Towers, S.K., Rozov, A., Buhl, E.H., Whittington, M.A. & Monyer, H. (2001) *Neuron* **31,** 487-95.
16.McBain, C.J. & Fisahn, A. (2001) *Nat Rev Neurosci* **2,** 11-23.
17.Traub, R.D., Pais, I., Bibbig, A., LeBeau, F.E., Buhl, E.H., Hormuzdi, S.G., Monyer, H. & Whittington, M.A. (2003) *Proc Natl Acad Sci USA* **100,** 1370-4.
18.Condorelli, D.F., Belluardo, N., Trovato-Salinaro, A. & Mudo, G. (2000) *Brain Res Brain Res Rev* **32,** 72-85.
19.Deans, M.R., Gibson, J.R., Sellitto, C., Connors, B.W. & Paul, D.L. (2001) *Neuron* **31**, 477-85.
20.Guldenagel, M., Ammermuller, J., Feigenspan, A., Teubner, B., Degen, J., Sohl, G., Willecke, K. & Weiler, R. (2001) *J Neurosci* **21,** 6036-44.
21.Deans, M.R., Volgyi, B., Goodenough, D.A., Bloomfield, S.A. & Paul, D.L. (2002) *Neuron* **36,** 703-12.
22.Landisman, C.E., Long, M.A., Beierlein, M., Deans, M.R., Paul, D.L. & Connors, B.W. (2002) *J Neurosci* **22,** 1002-9.
23.Long, M.A., Deans, M.R., Paul, D.L. & Connors, B.W. (2002) *J Neurosci* **22,** 10898-905.
24.Buhl, D.L., Harris, K.D., Hormuzdi, S.G., Monyer, H. & Buzsaki, G. (2003) *J Neurosci* **23,** 1013-8.
25.Panchin, Y., Kelmanson, I., Matz, M., Lukyanov, K., Usman, N. & Lukyanov, S. (2000) *Curr Biol* **10,** R473-4.
26.Wisden, W. & Morris, B.J. (2002) *Int Rev Neurobiol* **47,** 3-59.
27.Ernsberger, U., Patzke, H. & Rohrer, H. (1997) *Mech Dev* **68,** 115-26.
28.Groves, J.D. & Tanner, M.J. (1992) *J Biol Chem* **267,** 22163-70.
29.White, T.W., Deans, M.R., O'Brien, J., Al-Ubaidi, M.R., Goodenough, D.A., Ripps, H. & Bruzzone, R. (1999) *Eur J Neurosci* **11,** 1883-90.
30.Barrio, L.C., Suchyna, T., Bargiello, T., Xu, L.X., Roginski, R.S., Bennett, M.V. & Nicholson, B.J. (1991) *Proc Natl Acad Sci USA* **88,** 8410-4.
31.Bruzzone, R., Gomes, D., Denoyelle, E., Duval, N., Perea, J., Veronesi, V., Weil, D., Petit, C., Gabellec, M-M., D'Andrea, P. & White, T.W. (2001) *Cell Commun Adhes* **8,** 425-31.
32.White, T.W., Srinivas, M., Ripps, H., Trovato-Salinaro, A., Condorelli, D.F. & Bruzzone, R. (2002) *Am J Physiol Cell Physiol* **283,** C960-70.
33.Phelan, P. & Starich, T.A. (2001) *Bioessays* **23,** 388-96.
34.Johnston, M.F. & Ramon, F. (1981) *Biophys J* **39**, 115-7.
35.Verselis, V.K., Bennett, M.V. & Bargiello, T.A. (1991) *Biophys J* **59,** 114-26.
36.Kelmanson, I.V., Shagin, D.A., Usman, N., Matz, M.V., Lukyanov, S.A. & Panchin, Y.V. (2002) *Eur J Neurosci* **16,** 2475-6.
37.Paul, D.L., Ebihara, L., Takemoto, L.J., Swenson, K.I. & Goodenough, D.A. (1991) *J Cell Biol* **115,** 1077-89.
38.Stebbings, L.A., Todman, M.G., Phelan, P., Bacon, J.P. & Davies, J.A. (2000) *Mol Biol Cell* **11,** 2459-70.
39.Bruzzone, R., White, T.W. & Paul, D.L. (1996) *EurJ Biochem* **238,** 1-27.
40.Schmitz, D., Schuchmann, S., Fisahn, A., Draguhn, A., Buhl, E.H., Petrasch-Parwez, E., Dermietzel, R., Heinemann, U. & Traub, R.D. (2001) *Neuron* **31,** 831-40.
41.Traub, R.D., Bibbig, A., Piechotta, A., Draguhn, R. & Schmitz, D. (2001) *J Neurophysiol* **85,** 1246-56.
42.Ebihara, L., Beyer, E.C., Swenson, K.I., Paul, D.L. & Goodenough, D.A. (1989) *Science* **243,** 1194-5.
43.Srinivas, M., Rozental, R., Kojima, T., Dermietzel, R., Mehler, M., Condorelli, D.F., Kessler, J.A. & Spray, D.C. (1999) *J Neurosci* **19,** 9848-55.
44.Al-Ubaidi, M.R., White, T.W., Ripps, H., Poras, I., Avner, P., Gomes, D. & Bruzzone, R. (2000) *J Neurosci Res* **59,** 813-26.
45. Horwell *et al*., Bioorg. Med. Chem. **4:** 1573 (1996).
46. Liskamp *et al*., Recl. Trav. Chim. Pays-Bas **1**: 113 (1994).
47. Gante et *al*., Angew. Chem. Int. Ed. Engl. **33** : 1699 (1994).
48. Seebach et *al*., Helv. Chim. Acta **79 :** 913 (1996).

## Claims

1. Use of at least one neuronal channel-forming pannexin, or at least one biologically active fragment thereof, or at least one biologically active derivative thereof, for the manufacture of a drug for preventing and/or treating, in a mammal, a neurological disorder involving the hippocampal pyramidal cells.

2. Use of at least one neuronal channel-forming pannexin for *in vitro* diagnosing, in a mammal, a neurological disorder involving the hippocampal pyramidal cells.

3. The use according to claim 2, wherein said *in vitro* diagnostic comprises at least:
a) sequencing a pannexin gene in a mammal suspected to have a neurological disorder involving the hippocampal pyramidal cells; and
b) identifying at least one mutation responsible for the lack of production of pannexin or for the production of an inactive pannexin in said mammal.

4. The use according to any of claims 1 to 3, wherein said mammal is a human.

5. The use according to any of claims 1 to 4, wherein said neurological disorder is selected from epilepsy, schizophrenia, memory disorders, pain disorders, visual deficits, visual acuity, odor discrimination, and olfaction deficits.

6. Method for *in vitro* selecting a compound useful for preventing and/or treating, in a mammal, a neurological disorder involving the hippocampal pyramidal cells, said compound being capable of modifying the channel-forming ability of a pannexin, wherein said method comprises at least:
a) measuring the channel-forming ability of said pannexin in the absence of said compound (P0);
b) measuring the channel-forming ability of said pannexin in the presence of said compound (P1);
c) comparing P0 and P1; and
d) if P1 is significantly different from P0, selecting said compound.

7. Method for *in vitro* selecting a compound useful for preventing and/or treating, in a mammal, a neurological disorder involving the hippocampal pyramidal cells, said compound being capable of specifically modifying the channel-forming ability of a pannexin, without modifying the channel-forming ability of a connexin, wherein said method comprises at least:
a) measuring the channel-forming ability of each of said pannexin (P0) and said connexin (C0) in the absence of said compound;
b) measuring the channel-forming ability of each of said pannexin (P1) and said connexin (C1) in the presence of said compound;
c) comparing P0 and P1, and C0 and C1; and
d) if P1 is significantly different from P0, and if C1 is not significantly different from C0, selecting said compound.

8. The method according to claim 6 or 7, wherein, in step d), if P1 is significantly greater than P0, said compound is an agonist of said pannexin.

9. The method according to claim 6 or 7, wherein, in step d), if P1 is significantly lower than P0, said compound is an antagonist of said pannexin.

10. The method according to any of claims 6 to 9, further comprising purifying said compound.

11. The method according to any of claims 6 to 10, wherein said mammal is a human.

12. The method according to any of claims 6 to 11, wherein said neurological disorder is selected from epilepsy, schizophrenia, memory disorders, pain disorders, visual deficits, visual acuity, odor discrimination, and olfaction deficits.

13. An animal model for *in vivo* selecting a compound useful for preventing and/or treating, in a mammal, a neurological disorder involving the hippocampal pyramidal cells, wherein said compound is capable of modifying the channel-forming ability of a pannexin in said animal model.

14. The animal model according to claim 13, wherein at least one mutation is introduced in a pannexin gene of an animal, said mutation being responsible for the lack of production of pannexin, or for the production of an inactive pannexin, in said animal model.

15. The animal model according to claim 13 or 14, wherein said mammal is a human.

16. The animal model according to any of claims 13 to 15, wherein said neurological disorder is selected from epilepsy, schizophrenia, memory disorders, pain disorders, visual deficits, visual acuity, odor discrimination, and olfaction deficits.
